# EUROPEAN PATENT APPLICATION

(11) **EP 3 950 051 A1**
(43) Date of publication of application: **09.02.2022**
(21) Application number: 20782127.3
(22) Date of filing: 01.04.2020
(51) Int. Cl.: A61N 1/375, A61N 1/362

(54) **MEDICAL DEVICE AND FIXING MECHANISM FOR MEDICAL APPARATUS OF MEDICAL DEVICE**

(30) Priority: 02.04.2019 CN 201910262677
(71) Applicant: MicroPort Soaring CRM (Shanghai) Co., Ltd., Shanghai 201203 (CN)
(72) Inventor: WANG, Li, Shanghai 201203 (CN); CHENG, Zhijun, Shanghai 201203 (CN)
(74) Representative: Lavoix
(86) International application number: PCT/CN2020/082683
(87) International publication number: WO 2020/200222

(57) **Abstract**

A medical apparatus and a fixation mechanism for a medical device thereof are disclosed. The fixation mechanism allows the medical device to be easily fixed at a target site in a patient's body to better achieve the desired therapeutic effects intended for its implantation or intervention. The medical device may be a leadless pacemaker, and with the present invention, it can provide more physiological cardiac pacing. The fixation mechanism includes a holder (1) and a fixation member (4). The holder (1) extends in parallelism with the intended object, and the fixation member (4) is provided on an outer wall of the holder (1) and extends around the holder (1). One end of the fixation member (4) is connected to the outer wall of the holder (1), and the other end is spaced from the outer wall of the holder (1) by a gap. In practical use, the holder (1) is driven to rotate in the same direction as the extension of the fixation member (4), causing the fixation member (4) to rotate in the same way. As a result, the fixation member (4) directly penetrates tissue or a blood vessel wall that extends in parallelism with the holder (1). In this way, the medical device can be fixed to the atrial wall (12), the ventricular wall, the superior vena cava wall (14) or other blood vessel walls, or even to the interventricular septum (15) or the interatrial septum.

## Description

### TECHNICAL FIELD

The present invention relates to the field of medical instruments and, in particular, to a medical apparatus and a fixation mechanism for a medical device thereof.

### BACKGROUND

Since the advent in 1958, cardiac pacemakers have become a first-line treatment for bradycardia arrhythmia. With the advancements and innovations over the past more than half century, pacemakers have gradually evolved from the initial form of single leads for open chest implantation and ventricular pacing to the recent form of 2-3 leads for intravenous implantation and atrioventricular physiological pacing or even biventricular synchronous pacing. However, lead-related complications such as lead dislodgement, thrombosis, tricuspid regurgitation and infection have always been a handicap that not only interrupts the normal operation of pacemakers but also seriously threatens patients' health and lives and degrades their quality of life. For patients found with such lead-related complications, it is necessary to remove the leads as soon as possible. However, as such lead removal operations are associated with certain difficulties and risks, they usually have to be performed in large electrophysiology centers by dexterous surgeons, imposing great burden on resource utilization and surgeons' workload. In order to overcome these problems arising from the use of leads, "leadless" cardiac pacemakers have become a new focus of interest in the field of arrhythmia treatment.

Existing leadless pacemaker typically include a fixation mechanism that is separate from a pacing electrode and has a diameter equal to or less than an outer diameter of the pacemaker. The fixation mechanism is designed to penetrate tissue when rotated two or more revolutions, thus bringing the pacing electrode into contact with the tissue and achieving its fixation. In some embodiments, the fixation mechanism may include a plurality of hooks or projections provided around a distal end of the pacemaker. One or more of these fixation components may be configured to penetrate up to the endocardium and stay for their most part within the myocardium. There is a variant further including a circular ring-shaped fixation feature disposed at a proximal end of the leadless pacemaker, which can tether the leadless pacemaker within the right ventricle upon the failure of the distal fixation mechanism, preventing its entry into the blood circulation and consequent danger to the patient.

Obviously, all the existing leadless pacemakers are delivered to the right ventricular apex and then rotated to cause a helical feature at a distal end thereof to screw into the relatively thick apical myocardial tissue. However, since the atrial wall is very thin, fixing such leadless pacemakers to the atrial wall may lead to a significant risk of insecure fixation, atrial perforation, etc. Therefore, the existing leadless pacemakers are not suitable to be fixed to the atrial wall.

Since all these existing leadless pacemakers have to be fixed to the right ventricular apex, they are only suitable for univentricular pacing and incapable of dual-chamber pacing (DDD mode). Therefore, the existing leadless pacemakers tend to suffer from non-physiological pacing with atrioventricular desynchronization, limiting those who can truly benefit from the leadless pacing technology generally only to patients with chronic atrial fibrillation or at advanced ages.

### SUMMARY OF THE INVENTION

In view of the above, it is an object of the present invention to provide a medical apparatus and a fixation mechanism for a medical device thereof, which allows the medical device to be easily fixed at a target site in a patient's body to better achieve the desired therapeutic effects intended for its implantation or intervention.

In an aspect of the present invention, there is provided a fixation mechanism for a medical device, which is used to fix the medical device to an intended object. The fixation mechanism includes:
a holder extending in parallelism with the intended object when the medical device is fixed to the intended object; and
a fixation member arranged on an outer wall of the holder and extending around the holder, one end of the fixation member being connected to the outer wall of the holder, the other end of the fixation member being spaced from the outer wall of the holder by a gap,
wherein the fixation member is configured so that as a result of the holder being rotated in the same direction as the extension of the fixation member, the other end of the fixation member penetrates the intended object.

Optionally, the fixation member may extend helically over the outer wall of the holder.

Optionally, the holder may have a leading end toward which the fixation member extends.

Optionally, the fixation member may helically extend less than one turn circumferentially around the holder.

Optionally, the fixation member may helically extend less than or equal to one half turn circumferentially around the holder.

Optionally, a lead angle of the helical fixation member may range from 5° to 20°.

Optionally, the gap may be configured to receive therein part of the intended object when the fixation member has penetrated the intended object.

Optionally, the gap may have a width in the range of 0.1 -15.0 mm.

Optionally, the width of the gap may be in the range of 1.0-5.0 mm.

Optionally, the holder may be a cylinder, wherein the fixation member is curved, and wherein axial projections of the fixation member and the holder are concentric.

Optionally, as a result of the fixation member penetrating the intended object, the outer wall of the holder may be brought into contact with the intended object.

Optionally, a groove may be formed in the outer wall of the holder, in which the fixation member is stowed as a result of a force being applied thereto.

Optionally, the groove may extend helically.

Optionally, the other end of the fixation member may be provided thereat with a sharp tip.

Optionally, the fixation member may be provided in the form of a wire, a rod or a tab.

Optionally, the fixation member may have a circular or polygonal cross-sectional shape.

Optionally, the fixation member may be made of an elastic material.

Optionally, the fixation member may possess an electrical characteristic and may be further configured for exchange of information between the medical device and the intended object.

Optionally, the holder may have a leading end and an opposing trailing end, wherein the leading end is provided thereat with a spherical surface, and the trailing end is configured for detachable coupling to an external mechanism for driving the holder to rotate.

Optionally, a plurality of the fixation members may be arranged at different locations of the holder.

Optionally, the holder may be a hermetic enclosure configured to house therein at least some components of the medical device.

In another aspect of the present invention there is provided a medical apparatus including a medical device and the fixation mechanism. The medical device is provided on the fixation mechanism, and the fixation mechanism is configured to anchor to an intended object to fix the medical device at a target site for the intended object.

Optionally, the medical device may be an implantable medical device or an interventional medical device.

Optionally, the medical device may be a leadless pacing device including a control member and at least two electrodes,
wherein the control member is hermetically housed in the interior of the holder of the fixation mechanism, and wherein the at least two electrodes are all provided on the exterior of the holder of the fixation mechanism, coupled to the control member and configured to be brought into contact with the intended object.

Optionally, the medical device may be a leadless pacing device including a control member and at least two electrodes,
wherein the control member is hermetically housed in the interior of the holder of the fixation mechanism, wherein at least one electrode of the at least two electrodes is provided on the exterior of the holder of the fixation mechanism and coupled to the control member, and the rest of the at least two electrodes are provided on the fixation member of the fixation mechanism and coupled to the control member, and wherein each of the at least two electrodes is configured to be brought into contact with the intended object.

The medical apparatus and the fixation mechanism provided in the present invention have the following advantages:
First, the fixation mechanism is so configured that as a result of the holder being rotated, the fixation member arranged on the outer wall of the holder rotates in the same way and thus penetrate tissue or a blood vessel wall with which the holder is brought into contact. In this way, the medical device (e.g., the leadless pacemaker) can be easily fixed to the atrial wall, in a ventricle or at any other site. Moreover, with the fixation mechanism, the medical device can be fixed in the superior vena cava, the inferior vena cava or another blood vessel. In particular, the medical device can be fixed to the interatrial or interventricular septum to better achieve the desired therapeutic effects intended for its implantation or intervention. For the leadless pacemaker, fixing it to the interatrial or interventricular septum facilitates synchronized atrial or ventricular pacing and thus improves the heart's function. That is, the pacemaker can provide more physiological pacing to the heart.
Second, the fixation member helically extends over the outer circumference of the holder. This allows the fixation member to be more easily stowed into a delivery system while not being damaged. Moreover, it allows the fixation member to penetrate along a helical path, which causes less secondary damage to the patient, when compared to a circular path. In particular, the fixation member may extend less than one turn externally (circumferentially) about the holder. This allows a shorter length of the fixation member that penetrates tissue or a blood vessel wall and thus reduced damage thereto, as well as a smaller torque required to rotate the fixation member, which facilitates the physician's manual manipulation and shortens the required surgical time. More particularly, the gap between the fixation member and the outer wall of the holder may be configured so that when the fixation member pierces into tissue or a blood vessel wall, it will not lead to perforation. To this end, the gap is controlled, for example, within the range of 0.1-15.0 mm. This range can not only ensure shallow penetration of the fixation member that will not cause perforation, but can also ensure effective contact between the tissue and the outer wall of the holder.
Third, the fixation member can be stowed in the groove formed in the outer wall of the holder as a result of a force being applied thereto, allowing a reduced size for delivery, which will cause less damage. Moreover, the fixation member is essentially a structure with a sharp tip, which can be applied to any tissue wall thickness while ensuring effective penetration and fixation of the medical apparatus at a target site. Further, a desired penetration depth of the fixation member can be easily achieved by properly rotating the holder. Therefore, the fixation member can address various needs and allows higher flexibility in use.

### BRIEF DESCRIPTION OF THE DRAWINGS

The accompanying drawings are presented in order to provide a better understanding of the present invention without limiting it in any way. In these figures:
Fig. 1 is a structural schematic of a medical apparatus according to an embodiment of the present invention;
Fig. 2 is a schematic view of a fixation member being welded to a holder according to an embodiment of the present invention;
Fig. 3 is a schematic view of the fixation member extending while being spaced from the holder by a gap according to an embodiment of the present invention;
Fig. 4 is a schematic view of the fixation member being stowed in a groove according to an embodiment of the present invention;
Fig. 5 is a structural schematic of the medical apparatus provided with a plurality of the fixation members each in the form of a circular rod according to an embodiment of the present invention;
Fig. 6 is a structural schematic of a medical apparatus provided with one of the fixation member in the form of a tab according to an embodiment of the present invention;
Fig. 7 is a schematic view of the fixation member being provided thereon with an electrode according to an embodiment of the present invention;
Fig. 8 is an isometric cross-sectional view of a delivery system according to an embodiment of the present invention, showing only a distal end portion of the system;
Fig. 9 is an isometric cross-sectional view of a guide system according to an embodiment of the present invention, showing only a distal end portion of the system;
Fig. 10 is a schematic view of the medical apparatus with a proximal end being mated with the distal end of the delivery system according to an embodiment of the present invention;
Fig. 11 is a schematic view of a delivery sheath in the delivery system of Fig. 10 being pushed toward a distal end of the medical apparatus;
Fig. 12 is a schematic view of a guide system having been delivered into the superior vena cava according to an embodiment of the present invention;
Fig. 13 is a schematic view of a sharp tip of the fixation member in the medical apparatus having penetrated a vessel wall according to an embodiment of the present invention; and
Fig. 14 is a schematic view of the medical apparatus being fixed to the interventricular septum according to an embodiment of the present invention.

In these figures,
1-hermetic enclosure; 2-first electrode; 3-second electrode; 4-fixation member; 41-sharp tip; 5-prism; 6-groove; 8-delivery sheath; 9-rotating sheath; 10-expansion sheath; 11-guide sheath; 12-right atrium; 13-inferior vena cava; 14-superior vena cava; 15-interventricular septum.

### DETAILED DESCRIPTION

Objectives, features and advantages of the present invention will become more apparent upon reading the following more detailed description of the present invention, which is to be read in conjunction with the accompanying drawings. Note that the figures are provided in a very simplified form not necessarily drawn to exact scale for the only purpose of helping to explain the disclosed embodiments in a more convenient and clearer way.

The terms "leading", "trailing", "proximal" and "distal" will be used herein to describe relative orientations, relative positions or directions of components in a medical instrument or actions thereof with respect to one another, from the perspective of a physician who is operating the instrument. While not meant to be limiting, a "trailing" or "proximal" end is generally closer to the physician when in normal operation of the medical instrument, while a "leading" or "distal" end generally enters the body of a patient first.

As used herein and in the appended claims, the singular forms "a", "an", and "the" include plural references unless the context clearly dictates otherwise. As used herein and in the appended claims, the term "or" is generally employed in its sense including "and/or" unless the content clearly dictates otherwise.

The description hereinafter is set forth in the context of implanting a leadless pacemaker as an example of the medical device into the heart as an example of a target implantation site. However, the present invention is not so limited, as the medical device may alternatively be, for example, a cardiac defibrillator, an occluder, a valve or another type of medical device, and because the target implantation site may alternatively be, for example, the brain or another organ or region of the patient's body.

As described in the Background section, the existing leadless pacemakers have to be fixed to the right ventricular apex to provide only univentricular pacing because they rely on a helical feature arranged at the distal end, which can screw into the myocardium to fix the device. Although this fixation approach relying on the screwing action of the helical feature is feasible for the thick ventricular myocardial wall, in particular for the right ventricular apex, it would be problematic for the atrial wall due to a relatively small myocardial thickness thereof. In the latter case, insecure fixation, atrial perforation and other issues tend to arise. Therefore, the helical feature can be only used to fix a leadless pacemaker to the relatively thick ventricular myocardial wall but not to the thin atrial myocardial wall. Moreover, the helical feature is typically arranged at the distal end of the pacemaker in parallelism and coaxiality with the pacemaker. For this reason, the pacemaker has to be fixed in a perpendicular orientation and thus to have a high profile, making it difficult to be deployed at a target site with a limited accommodation space, such as a vessel wall or the interventricular septum. Therefore, existing leadless pacemakers are not suitable to be fixed to a vessel wall or the interventricular septum. Instead, they are usually fixed to the right ventricular apex that allows easiest fixation, making the right ventricular apex the most common pacing site at present.

However, studies have shown that long-term pacing from the right ventricular apex, even in the dual-chamber DDD mode, is not physiological, and too much such spacing may cause myocardial reconstruction and cardiac insufficiency. Specifically, stimuli from the right ventricular apex tend to cause non-physiological pacing with atrioventricular desynchronization featuring asynchronous ventricular contraction, and long-term right ventricular apex pacing may lead to structural and electrical reconstruction of the ventricular myocardium and eventually impair the heart's function. Therefore, the right ventricular apex is not an ideal pacing site. On the other hand, pacing from the superior vena cava near the sinoatrial node or from a septum near the atrioventricular bundle is considered to be more physiological and have less adverse impact on the heart's function.

To this end, the present invention proposes a fixation mechanism of a medical device, which can fix the medical device not only to the atrial or ventricular wall but also to the superior vena cava wall, or even to the interatrial or interventricular septum to allow it to better achieve the desired therapeutic effects intended for the device's implantation or intervention while addressing the needs of different patients. The medical device proposed in the present invention may be an implantable or interventional medical device. Examples of the implantable medical device may include, but are not limited to, a leadless pacemaker. Examples of the interventional medical device may include, but are not limited to, a capsule endoscope or another medical device in need of fixation to one of the above sites.

In the present invention, the fixation mechanism of the medical device specifically includes a holder and a fixation member. The holder extends in parallelism with an intended object, which may be a vessel wall, the atrial wall or the ventricular wall. Alternatively, it may also be the interventricular or interatrial septal wall. Here, the term "parallelism" refers not only to absolute parallelism but also to parallelism with a certain degree of tilt. The fixation member is arranged on an outer wall of the holder and extends around the holder in such a manner that one end of the fixation member is connected to the outer wall of the holder and the other end is spaced apart from the outer wall of the holder by a gap. When the fixation member penetrates body tissue or a blood vessel wall, part of the tissue or vessel wall may be clamped in the gap. In practical use, the holder may be rotated by a delivery system in the same direction as the extension of the fixation member so that a leading portion (i.e., a portion at the other end) of the fixation member pierce into the tissue or vessel wall. It is to be understood that the extension direction of the fixation member refers to the direction from its junction with the holder to the other end. Preferably, the fixation member extends toward a leading end of the holder.

Apparently, according to the present invention, arranging the fixation member around the holder and orienting the holder in parallelism with a blood vessel wall or a tissue wall enables the fixation mechanism to fix the medical device to the wall of the superior vena cava or another blood vessel, or to the atrial or ventricular wall, or to the interventricular or interatrial septum, making the medical device applicable to more patients. When the medical device is a leadless pacemaker, it may be fixed to the superior vena cava wall to enable atrial pacing. Moreover, as the sinoatrial node is close to the superior vena cava, the pacemaker may be fixed in the vicinity of the sinoatrial node for sensing and/or pacing. In this case, in response to any abnormal activity in the sinoatrial node, an electrical impulse delivered from an electrode of the pacemaker can provide a stimulus that is more consistent with the normal activation sequence of the heart, enabling more physiological pacing of the heart by the pacemaker. When fixed to the interventricular septum, the leadless pacemaker is capable of more physiological simultaneous pacing of the left and right ventricles.

In case of the medical device being implemented as a leadless pacemaker, in order to enable an electrode thereof to receive an electrical signal from the heart or to apply a pacing signal to tissue, the outer wall of the holder is preferably brought into contact with the tissue. Of course, when the medical device is implemented as a different medical device such as a capsule endoscope, it may not be necessarily brought into contact with tissue unless such contact is required to enable transfer of energy or information.

In embodiments of the present invention, the fixation member preferably extends helically over the outer wall of the holder. This helical extension allows the fixation member to be more easily stowed into a delivery system while not being damaged. Moreover, it allows the fixation member to penetrate along a helical path, which causes less secondary damage to the patient, when compared to a circular path. The fixation member extends preferably less than one turn, more preferably, one half turn or less, externally about the holder (i.e., circumferentially with respect thereto, as seen when projected onto a plane laterally crossing the holder). This allows a shorter length of the fixation member that penetrates tissue or a blood vessel wall and thus reduced damage thereto, as well as a smaller torque required to rotate the fixation member, which facilitates the physician's manual manipulation and shortens the required surgical time. Moreover, the shortened penetration length of the fixation member can avoid the risk of perforation when it is used to fix the medical device, for example, to the atrial wall. Further, the fixation member extends helically forward essentially in the same direction as advancement of the delivery system toward a distal end of the medical apparatus. In this way, with the advancement of the delivery system, the fixation member can be compliantly compressed onto the holder, without hindering sheathing of the medical apparatus. Additionally, this allows the medical apparatus to be delivered in a more compact configuration. More preferably, a lead angle of the helical fixation member ranges from 5° to 20°. Such a lead angle in this range facilitates easy stowing of the fixation member and more secure fixation. It would be understood that an excessive lead angle of the helix may lead to difficult penetration of tissue or a blood vessel wall and easy dislodgement even after the penetration. On the other hand, a too small lead angle of the helix may lead to difficult compression of the fixation member, a large size of the delivery system or easy damage to the fixation member.

In another embodiment of the present invention, in order to further avoid the risk of perforation, the gap left between the leading end of the fixation member and the outer wall of the holder is configured so that when the fixation member pierces into tissue or a blood vessel wall, it will not lead to perforation. To this end, the gap is controlled preferably within the range of 0.1-15.0 mm, more preferably, within the range of 1.0-5.0 mm. Such a range can ensure shallow penetration of the fixation member that will not cause perforation. Of course, the gap is not limited to any particular value, and those skilled in the art can properly determine it based on a diameter of the pacemaker, an intended tissue thickness and a thickness of the fixation member, as long as a desired portion of the tissue is clamped in the gap.

The medical apparatus and the fixation mechanism provided in the present invention will be described by way of specific embodiments with reference to the accompanying drawings. While the following description is given in the context of a leadless pacemaker as an example of the medical device that can be easily fixed by the fixation mechanism, the present invention is in no way so limited.

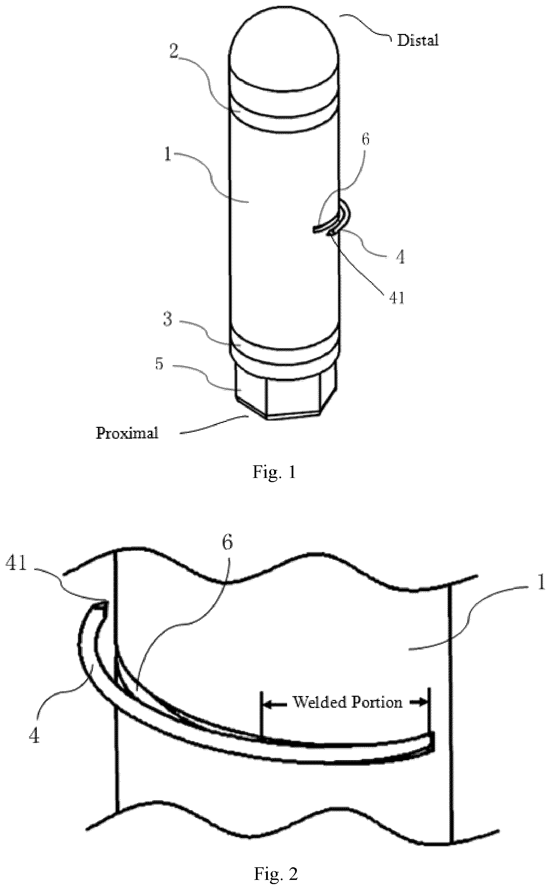

Reference is now made to Fig. 1, a structural schematic of a medical apparatus according to an embodiment of the present invention. The medical apparatus is a cardiac pacing apparatus including a fixation mechanism and a leadless pacemaker provided on the fixation mechanism. The fixation mechanism is used to anchor and fix the leadless pacemaker to a target site in a patient's body.

The leadless pacemaker includes a hermetic enclosure 1, a first electrode 2, a second electrode 3 and a control member (not shown). The control member is arranged within the hermetic enclosure 1 and includes, among others, an impulse generator, a communication module, a battery and a processor. The impulse generator is configured to generate a pacing impulse and transmit it to the second electrode 3 configured for, e.g., pace making (i.e., a pacing electrode). The second electrode 3 is further configured to deliver the pacing impulse to the myocardium to provoke a contraction of the myocardium. The communication module may utilize a wireless communication technique to establish a wireless connection for data exchange with an external device. In some embodiments, the communication module may receive a command from an external programmer and transmit it to the processor, the processor may then modulate the impulse generator to make changes in the output impulse. As a key component of the leadless pacemaker, the processor is configured to perform various functions including data storage, data output and scheduling between the various modules. The first electrode 2 may be, for example, a sensing electrode for transmitting an electrical signal representative of atrial or ventricular activity to the processor, which may then perform a computation on the electrical signal and modulate the impulse generator based on the computation results so that a desired change occurs in the output impulse. The battery is configured to power and ensure normal operation of the impulse generator, the communication module, the processor and other electronic components necessary for the operation of the cardiac pacemaker.

In this embodiment, the second electrode 3 and first electrode 2 may be coupled individually to the control member and to each other. During operation of the leadless pacemaker, the control member, the second electrode 3 and first electrode 2 may together form a loop that enables cardiac pacing or sensing of the leadless pacemaker. In other embodiments, more than two electrodes may be included to impart additional capabilities to the leadless pacemaker. For the sake of brevity, the following description is given in the context of two electrodes being included, i.e., the second electrode 3 and first electrode 2. A person skilled in the art would be able to adapt the following description to cases with more than two electrodes by making appropriate modifications in details.

The present invention is not limited to any particular shape of the hermetic enclosure 1, and in addition to the cylindrical shape as shown, other examples of the shape of the hermetic enclosure may include a rectangular parallelepiped, a prism and other polyhedrons. It is to be understood that the hermetic enclosure 1 is provided as the aforementioned holder. That is, the package enclosure of the leadless pacemaker itself serves as a carrier for the fixation member. However, the present invention is not so limited, if the medical device is not housed in an enclosure, a separate holder may be provided to support the fixation member, and the medical device may be hermetically provided either outside or inside the holder.

In one embodiment, both the first electrode 2 and second electrode 3 are arranged on the hermetic enclosure 1, but the present invention is not limited to any particular locations of the hermetic enclosure 1 where the electrodes are arranged, as long as they can be brought into contact with the intended tissue or vessel wall. Optionally, the first electrode 2 may be provided at a leading end of the hermetic enclosure 1, while the second electrode 3 may be provided at a trailing end of the hermetic enclosure 1. This allows a simple structure that can be easily fabricated and processed. Alternatively, either one of the first electrode 2 and second electrode 3 may be provided on the fixation member of the fixation mechanism. For example, the first electrode 2 may be provided on the hermetic enclosure 1, with the second electrode 3 being arranged on the fixation member. Alternatively, the second electrode 3 may be provided on the hermetic enclosure 1, with the first electrode 2 being arranged on the fixation member. This arrangement allows the electrode to be inserted in or brought into contact with the tissue when the fixation member has penetrated the tissue to provide improved pacing or sensing performance.

The fixation mechanism according to this embodiment is further provided with the fixation member 4, the fixation member 4 is arranged on an outer wall of the hermetic enclosure 1 so as to extend about an axis of the hermetic enclosure 1. Moreover, the axis of the hermetic enclosure 1 extends in parallelism with the intended tissue or vessel wall. That is, the hermetic enclosure 1 extends in parallelism with the tissue or vessel wall. A trailing portion of the fixation member 4 is connected to the outer wall of the hermetic enclosure 1, and the rest extends while being spaced from the outer wall of the hermetic enclosure 1. The spaced portion can penetrate and anchor in the tissue or vessel wall. In preferred embodiments of the present invention, the fixation member 4 helically extends over the outer wall of the hermetic enclosure 1 in a direction from the proximal to distal end of the medical apparatus. Preferably, the fixation member 4 helically extends less than one turn (over the hermetic enclosure 1), e.g., one half or one quarter turn, depending on the actual need. The extending length is designed to avoid dislodgement of the fixation member 4 and perforation of the tissue or vessel wall.

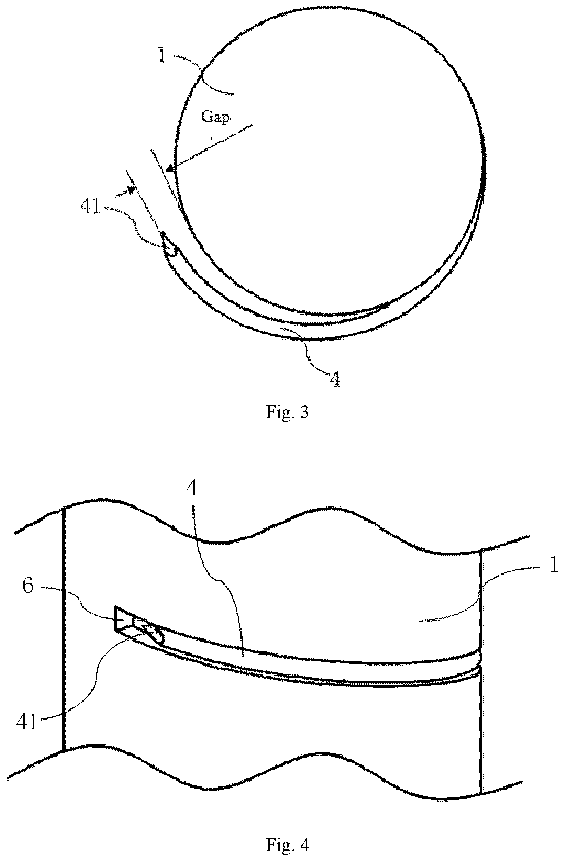

Reference is now made to Fig. 2, a schematic view of the fixation member being welded to the holder according to an embodiment of the present invention. The fixation member 4 may be configured as a relatively short curved wire or rod, which extends helically upward from a trailing to leading end thereof about the axis of the hermetic enclosure 1. A trailing portion of the fixation member 4 is welded to the hermetic enclosure 1 (i.e., the "Welded Portion" labeled in Fig. 2), and the present invention is not limited to any particular length of this trailing portion. Reference is additionally made to Fig. 3. At the leading end of the fixation member 4, a sharp tip 41 may be provided, which may guide the fixation member 4 to smoothly penetrate the tissue or vessel wall. In addition, a small gap may be left between the fixation member 4 and the outer wall of the hermetic enclosure 1. Specifically, the sharp tip 41 may be spaced from the outer wall of the hermetic enclosure 1 by a distance controlled within the range of 0.1-15.0 mm or 1.0-5.0 mm. In this way, the fixation member 4 may penetrate to a shallow depth in the tissue or vessel wall while allowing part of the tissue to be clamped between the fixation member and the outer wall of the hermetic enclosure so that the outer wall of the hermetic enclosure is brought into direct contact with the tissue.

With continued reference to Fig. 3, the hermetic enclosure 1 is preferably configured as a cylinder, which allows better contact with the tissue or vessel wall. Moreover, the fixation member 4 is preferably configured as such a curved component that axial projections of the fixation member 4 and hermetic enclosure 1 are concentric. This ensures more effective penetration of the fixation member 4 with a shallow depth in the tissue or vessel wall while providing a uniform force distribution across the fixation member 4, which makes the penetration of the tissue or vessel wall easier.

Referring to Fig. 4, in the outer wall of the hermetic enclosure 1, a groove 6 is preferably formed, which is preferably not brought into communication with the inside of the enclosure to ensure hermeticity thereof. In practice, in order to load the medical apparatus, a delivery system may be advanced toward the distal end of the medical apparatus. The fixation member 4 may be then completely stowed in the groove 6 under the action of a delivery sheath, resulting in a reduced size for delivery. In order to enable the complete stowing of the helically extending fixation member 4, the groove 6 is preferred to also extend helically. In practical implementations, the trailing portion of the fixation member 4 may also be secured in the groove 6, for example, by welding as described above, or otherwise such as by adhesive bonding. The groove 6 may have a width and length, which allow the fixation member 4 to be completely stowed therein. According to embodiments of the present invention, the fixation member 4 is preferably made of a shape memory alloy with high elasticity. Examples of the alloy may include, but are not limited to, nickel-titanium alloys. The high elasticity of the alloy enables it to deform in a desirable way, thus facilitating the reception of the fixation member 4. Further, once not be confined by a delivery system anymore, the fixation member 4 can immediately restore its original shape.

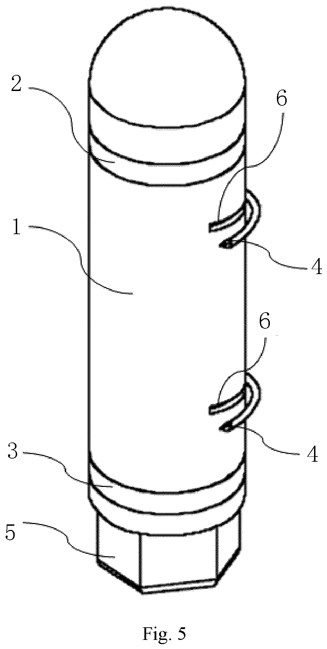

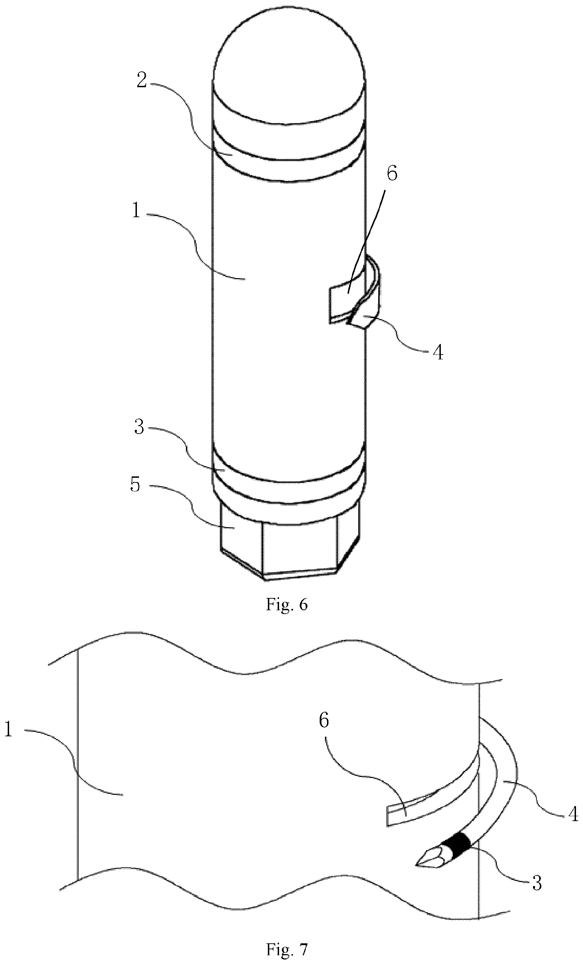

Reference is additionally made to Fig. 5. A plurality of the fixation members 4 may be provided. As a non-limiting example, two of the fixation member 4 may be provided. The plurality of fixation members 4 may be provided at different locations of the hermetic enclosure 1. For example, they may be spaced from one another on the same circumference. Alternatively, they may be aligned with or staggered from one another on different circumferences. Here, by the phrase "aligned with", it is meant to mean that the plurality of fixation members 4 are arranged on different circumferences so that their axial projections coincide with one another. The fixation member 4 may be in the form either of a rod or wire as shown in Fig. 1, or of a tab as shown in Fig. 6. However, the present invention is not limited to any particular cross-sectional shape of the fixation member 4, because it may be either circular or polygonal such as triangular, rectangular or square.

Referring further to Fig. 7, the fixation member 4 may be configured itself with an electrical characteristic. For example, the fixation member 4 may be connected to the control member, with the pacing or sensing electrode being provided on the fixation member 4. With this arrangement, once the fixation member 4 is brought into contact with tissue, a signal may be applied to or received from the tissue. Thus, even when the hermetic enclosure is not contact with the tissue, exchange of information (including signals) can be accomplished by the contact between the fixation member and the tissue.

With continued reference to Fig. 1, the leading end of the hermetic enclosure 1 is preferably a smooth spherical surface, which facilitates advancement of the medical apparatus with less damage caused to tissue, thus providing safer, more reliable cardiac pacing. The trailing end of the hermetic enclosure 1 opposing the leading end is configured for detachable coupling to a delivery system, which is an external mechanism for driving the medical apparatus to rotate. Optionally, the trailing end may be a prism 5.

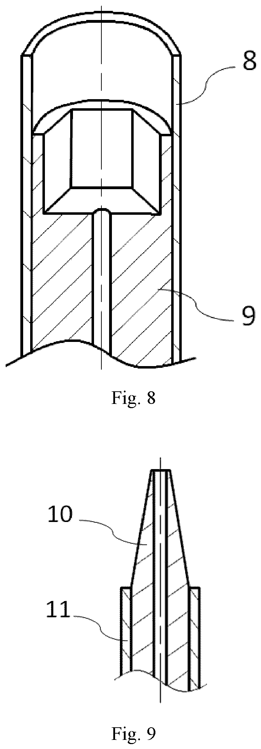

Further, as shown in Fig. 8, in embodiments of the present invention, there is also provided a delivery system for delivering and tuning the medical apparatus as discussed above to an optimal pacing site. The delivery system includes a delivery sheath 8 and a rotating sheath 9. The rotating sheath 9 is nested in a central bore of the delivery sheath 8. The delivery sheath 8 is axially movable and circumferentially rotatable relative to the rotating sheath 9. The delivery sheath 8 is essentially a circular tubular structure, and a polygonal blind hole for mating with the prism 5 at the trailing end of the hermetic enclosure 1 is provided at a distal end of the rotating sheath 9. However, in alternative embodiments, the distal end of the rotating sheath 9 may be detachably coupled to the trailing end of the hermetic enclosure 1 by threading. However, the present invention is not limited to any particular method for detachable coupling between the rotating sheath 9 and the hermetic enclosure 1.

As shown in Fig. 9, in embodiments of the present invention, there is also provided a guide system for setting up a delivery path in a patient's body. The guide system includes an expansion sheath 10 and a guide sheath 11, both of the expansion sheath 10 and guide sheath 11 may be made of a polymeric material. In practical use, the expansion sheath 10 may be inserted in a central bore of the guide sheath 11 in such a manner that it is movable in the central bore of the guide sheath 11. The delivery system may be delivered by the guide system to a target site through the guide sheath 11.

The delivery of the medical apparatus will be further described below in the context of a leadless pacemaker being fixed to the superior vena cava wall as an example.

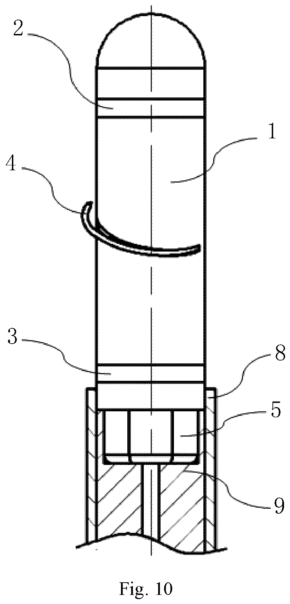

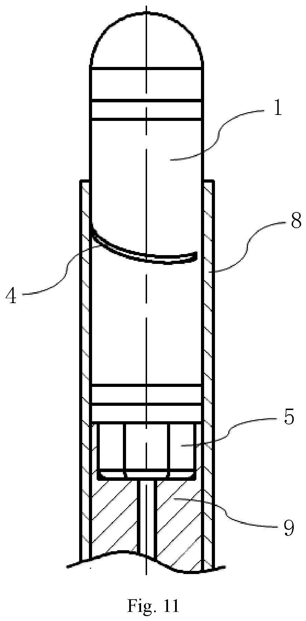

Prior to the implantation, the medical apparatus is loaded into the delivery system. As shown in Fig. 10, the prism 5 at the trailing end of the hermetic enclosure 1 is inserted into the polygonal blind hole of the rotating sheath 9, and the hermetic enclosure 1 is placed into the central bore of the delivery sheath 8. Subsequently, as shown in Fig. 11, with the rotating sheath 9 being held stationary, the delivery sheath 8 is pushed toward the distal end of the medical apparatus, so that the medical apparatus is at least partially received in the delivery sheath 8, and the fixation member 4 is received in the groove 6.

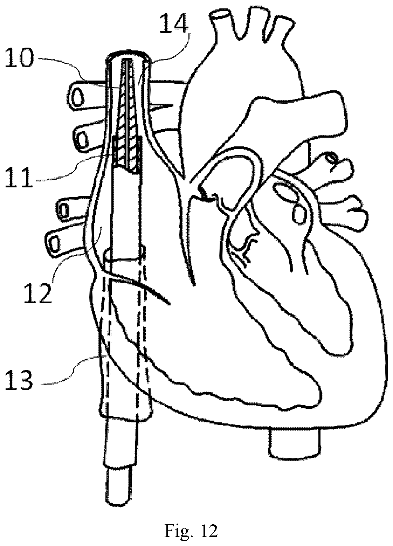

After that, the guide system is delivered into the patient's body. As shown in Fig. 12, the expansion sheath 10 and the guide sheath 11 are introduced via a femoral vein and advanced through the inferior vena cava 13 and the right atrium 12 into the superior vena cava 14. The expansion sheath 10 is then withdrawn, with the guide sheath 11 being retained there.

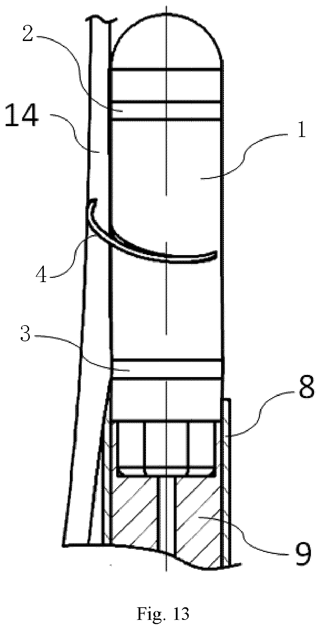

The delivery system with the medical apparatus being loaded therein is delivered into the superior vena cava through the guide sheath 11. Specifically, the medical apparatus and the delivery system in the configuration shown in Fig. 11 are both inserted into the guide sheath 11 and then advanced to a distal end of the guide sheath 11. The delivery sheath 8 is then retracted to the position shown in Fig. 10 so that the fixation member is exposed therefrom. A trailing end of the delivery sheath 8 is manipulated so that the second electrode 3 and first electrode 2 are brought into contact with the wall of the superior vena cava, and relevant electrical parameters are then measured. If the measured values of the parameters are satisfactory, the rotating sheath 9 is rotated in the same direction as the extension of the fixation member 4, causing the medical apparatus to rotate in the same way until the sharp tip 41 of the fixation member 4 penetrates the superior vena cava wall, as shown in Fig. 13.

Finally, the guide sheath 11, the delivery sheath 8 and the rotating sheath 9 are all withdrawn from the patient's body.

Of course, according to the present invention, the medical apparatus is allowed to be retrieved when its release is found to be unsatisfactory prior to the withdrawal of the guide sheath 11, the delivery sheath 8 and the rotating sheath 9. The retrieval can be accomplished simply by removing the fixation member 4 from the superior vena cava wall through rotating the rotating sheath 9 in the opposite direction. The medical apparatus is then reloaded in the delivery sheath 8 by pushing the delivery sheath 8 distally, and can be again released at a later time.

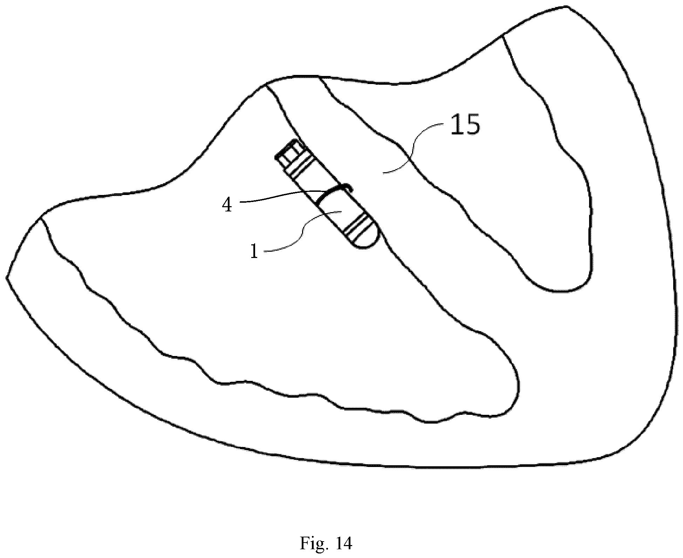

Alternatively, the guide sheath 11 may be introduced via a femoral vein and advanced through the inferior vena cava 13 and the right atrium 12 to a location near the interventricular septum 15 in the right ventricle, and a bendable delivery sheath may be then used to fix the leadless pacemaker to the interventricular septum 15, as shown in Fig. 14.

In summary, according to embodiments of the present invention, the medical apparatus is preferably fixed using the fixation member that extends helically from a proximal to distal end thereof. This fixation member can easily fix the medical device and can be easily stowed while not causing damage to tissue. Moreover, the fixation member is essentially a structure with a sharp tip, which can be applied to any tissue wall thickness while ensuring effective penetration and fixation of the medical apparatus at a target site. Additionally, a desired penetration depth of the fixation member can be easily achieved by properly rotating the hermetic enclosure. Therefore, the fixation member can address various needs and allows higher flexibility in use. With the fixation member, the medical apparatus can be fixed to the ventricular or atrial wall to provide physiological dual-chamber pacing with atrioventricular synchronization. It can also be fixed to the interventricular septum to provide synchronized pacing of the left and right ventricles. It can also be fixed to the interatrial septum to provide synchronized pacing of the left and right atria. It can also be fixed to the superior vena cava wall to provide atrial pacing. It can also be fixed in the vicinity of the sinoatrial node to provide more physiological cardiac pacing.

The description presented above is merely that of a few preferred embodiments of the present invention and is not intended to limit the scope thereof in any sense. Any and all changes and modifications made by those of ordinary skill in the art based on the above teachings fall within the scope as defined in the appended claims.

## Claims

1. A fixation mechanism for a medical device, which is used to fix the medical device to an intended object, the fixation mechanism comprising:
a holder extending in a direction parallel to a direction in which the intended object extends when the medical device is fixed to the intended object; and
a fixation member arranged on an outer wall of the holder and extending around the holder, one end of the fixation member being connected to the outer wall of the holder, and the other end of the fixation member being spaced from the outer wall of the holder by a gap,
wherein the fixation member is configured so that as a result of the holder being rotated along an extending direction of the fixation member, the other end of the fixation member is inserted into the intended object.

2. The fixation mechanism for a medical device of claim 1, wherein the fixation member helically extends over the outer wall of the holder.

3. The fixation mechanism for a medical device of claim 1 or 2, wherein the holder has a leading end, and the fixation member extending toward the leading end of the holder.

4. The fixation mechanism for a medical device of claim 2, wherein the fixation member helically extends less than one turn circumferentially around the holder.

5. The fixation mechanism for a medical device of claim 4, wherein the fixation member helically extends less than or equal to one half turn circumferentially around the holder.

6. The fixation mechanism for a medical device of claim 4 or 5, wherein a lead angle of the helical fixation member ranges from 5° to 20°.

7. The fixation mechanism for a medical device of claim 1 or 2, wherein the gap is configured to receive therein part of the intended object when the fixation member is inserted into the intended object.

8. The fixation mechanism for a medical device of claim 7, wherein the gap has a width in the range of 0.1 -15.0 mm.

9. The fixation mechanism for a medical device of claim 8, wherein the width of the gap is in the range of 1.0-5.0 mm.

10. The fixation mechanism for a medical device of claim 1 or 2, wherein when the fixation member is fixed to the intended object, the outer wall of the holder is brought into contact with the intended object.

11. The fixation mechanism for a medical device of claim 1 or 2, wherein a groove is formed in the outer wall of the holder, in which the fixation member is received as a result of a force being applied to the fixation member.

12. The fixation mechanism for a medical device of claim 11, wherein the groove extends helically.

13. The fixation mechanism for a medical device of claim 1 or 2, wherein the fixation member is provided in the form of a wire, a rod or a tab.

14. The fixation mechanism for a medical device of claim 1 or 2, wherein the fixation member is formed of an elastic material.

15. The fixation mechanism for a medical device of claim 1 or 2, wherein the fixation member possesses an electrical characteristic and is further configured for exchange of information between the medical device and the intended object.

16. The fixation mechanism for a medical device of claim 1 or 2, wherein the holder has a leading end and a trailing end opposite to the leading end, the leading end provided thereat with a spherical surface, the trailing end configured for detachable coupling to an external mechanism.

17. The fixation mechanism for a medical device of claim 1 or 2, wherein the holder is a hermetic enclosure configured to house at least part of components of the medical device in the hermetic enclosure.

18. A medical apparatus comprising a medical device and the fixation mechanism of any one of claim 1 to 17, the medical device provided on the fixation mechanism, the fixation mechanism configured to anchor to an intended object to fix the medical device at a target site for the intended object.

19. The medical apparatus of claim 18, wherein the medical device is an implantable medical device or an interventional medical device.

20. The medical apparatus of claim 19, wherein the medical device is a leadless pacing device comprising a control member and at least two electrodes,
the control member hermetically housed in an interior of the holder of the fixation mechanism, the at least two electrodes all provided on an exterior of the holder and all coupled to the control member, each of the at least two electrodes configured to be brought into contact with the intended object.

21. The medical apparatus of claim 19, wherein the medical device is a leadless pacing device comprising a control member and at least two electrodes,
the control member hermetically housed in an interior of the holder of the fixation mechanism, at least one electrode of the at least two electrodes provided on the holder of the fixation mechanism and coupled to the control member, the rest of the at least two electrodes provided on the fixation member of the fixation mechanism and coupled to the control member, each of the at least two electrodes configured to be brought into contact with the intended object.
